(19) Europäisches Patentamt
European Patent Office
Office européen des brevets

(11) Veröffentlichungsnummer : **0 455 119 A2**

# (12) EUROPÄISCHE PATENTANMELDUNG

(21) Anmeldenummer : **91106567.0**

(22) Anmeldetag : **24.04.91**

(51) Int. Cl.[5] : **C07C 403/24,** C07C 403/12, C07F 9/54

(30) Priorität : **03.05.90 DE 4014203**

(43) Veröffentlichungstag der Anmeldung : **06.11.91 Patentblatt 91/45**

(84) Benannte Vertragsstaaten : **CH DE FR GB LI NL**

(71) Anmelder : **BASF Aktiengesellschaft**
**Carl-Bosch-Strasse 38**
**W-6700 Ludwigshafen (DE)**

(72) Erfinder : **Ernst, Hansgeorg, Dr.**
**Bussardweg 62**
**W-6720 Speyer (DE)**
Erfinder : **Paust, Joachim, Dr.**
**Ringstrasse 3**
**W-6708 Neuhofen (DE)**
Erfinder : **Hoffmann, Werner, Dr.**
**Ringstrasse 11**
**W-6708 Neuhofen (DE)**

(54) **Verfahren zur Herstellung von Canthaxanthin und Astaxanthin.**

(57) Verfahren zur Herstellung von Canthaxanthin (Ia) und Astaxanthin (Ib) der allgemeinen Formel I

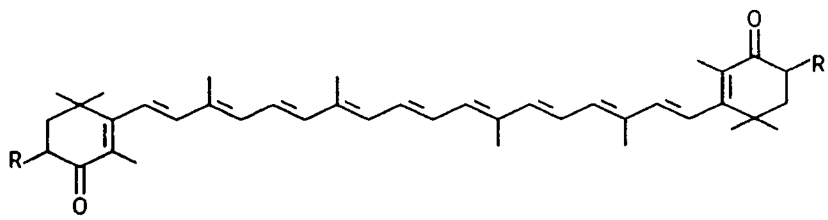

in der R für H (a) oder OH (b) steht, das dadurch gekennzeichnet ist, daß man einen tertiären Alkohol der allgemeinen Formel II

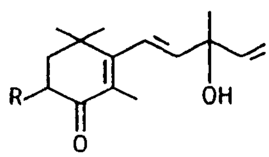

(II)

in der R für H (a) oder OH (b) steht, mit Trifluoressigsäure umsetzt, das erhaltene neue Trifluoracetat der allgemeinen Formel III

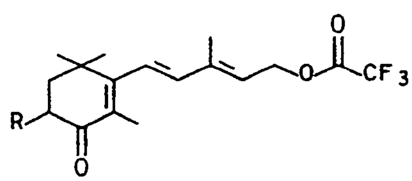

(III)

mit Triphenylphosphin umsetzt, das erhaltene neue Triphenylphosphoniumtrifluoracetat der allgemeinen Formel IV

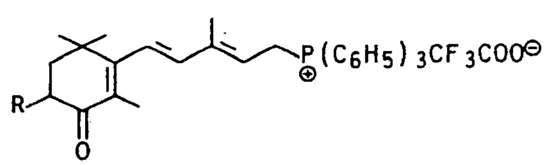

(IV)

mit 2,7-Dimethyl-2,4,6-octatrien-1,8-dial unter den Bedingungen einer Wittigsynthese umsetzt. Darüber

EP 0 455 119 A2

hinaus betrifft die Erfindung die neuen Trifluoracetate der Formel III und die entsprechenden Triphenylphosphoniumtrifluoracetate der Formel IV.

Die Erfindung betrifft ein neues Verfahren zur Herstellung der begehrten Carotinoide Canthaxanthin (Ia) und Astaxanthin (Ib).

Ein mehrfach beschriebenes Synthesekonzept für diese beiden Carotinoide beruht auf der zweifachen Wittig-Kondensation eines entsprechenden $C_{15}$-Phosphoniumsalzes mit dem symmetrischen $C_{10}$-Dialdehyd 2,7-Dimethyl-2,4,6-octatrien-1,8-dial.

Nach dem Stand der Technik werden als $C_{15}$-Phosphoniumsalze die Halogenide der nachstehenden Formel eingesetzt, wobei als Anion des Phosphoniumsalzes insbesondere das Bromid Verwendung findet.

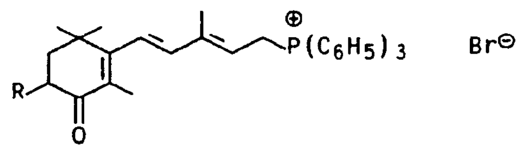

R = H (a)
R = OH (b)

Die nach dem oben geschilderten Syntheseprinzip $C_{15}$+$C_{10}$+$C_{15}$ als $C_{15}$-Bausteine für Canthaxanthin bzw. Astaxanthin geeigneten Phosphoniumsalze werden hierbei durch Umsetzung der entsprechenden Allylbromide der Formel

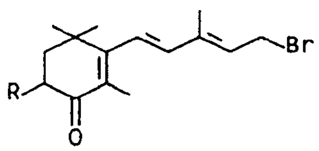

R = H (a)
R = OH (b)

mit Triphenylphosphin hergestellt.

Ein Zugang zu den genannten Allylbromiden besteht in der halogenierenden Allylumlagerung der tertiären Alkohole der Formel II

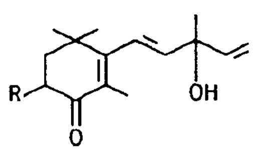

(II),

in der R für H (a) oder OH (b) steht.

Dieser in der Literatur mehrfach beschriebene Syntheseweg weist bei der praktischen Durchführung im Hinblick auf verfahrenstechnische Erfordernisse und Wirtschaftlichkeit schwerwiegende Nachteile auf.

So muß beispielsweise die Umsetzung des tertiären Alkohols der Formel IIb zu dem entsprechenden Allylbromid bei tiefer Temperatur (gemäß EP 101 597 bei -20°C bis -10°C; EP 5749 bei +5°C und gemäß Helv. chim. acta 64 (1981), Seite 2430 bei 0°C) durch Reaktion mit 63 %iger wäßriger Bromwasserstoffsäure durchgeführt werden, wobei ein erheblicher überschuß an Bromwasserstoff (2,5-4 Äquivalente) benötigt wird. Gemäß Angaben in Helv. chim. acta 64 (1981), Seiten 2430 und 2440, muß die Umsetzung bei tiefer Temperatur und sehr schnell durchgeführt werden, um eine Zersetzung des nicht sehr stabilen Allylbromids zu vermeiden und die Umlagerung des α-Hydroxyketons ins Diosphenol hintan zu halten.

Bei der Weiterverarbeitung des Allylbromids muß zum Abfangen von Säurespuren außerdem mehrmals Butylenoxid zugesetzt werden.

Zur Herstellung von Canthaxanthin wird nach dem Stand der Technik der tertiäre Alkohol der Formel IIa durch Behandeln mit Phosphortribromid in einem inerten organischen Lösungsmittel in das entsprechende Allylbromid überführt, aus dem durch Umsetzung mit Triphenylphosphin das entsprechende Phosphoniumsalz erhalten (DE-OS 2 801 908) wird. Auch hierbei muß mit einem erheblichen überschuß (≧ 3 Äquivalente) an dem teueren Halogenierungsmittel gearbeitet werden. Alternativ zur Bromierung mit Phosphortribromid und anschließender Umsetzung mit Triphenylphosphin kann man das Triphenylphosphoniumbromid auch direkt aus dem tertiären Alkohol der Formel IIa durch Reaktion mit Triphenylphosphin-Hydrobromid herstellen (vgl. DE-OS 2 801 908). Dieses Reagens muß allerdings in einer zusätzlichen Stufe aus Triphenylphosphin und HBr hergestellt werden und muß gleichfalls in beträchtlichem überschuß eingesetzt werden.

In J. Org. Chem. 47 (1982) Seite 2133, wird als beste Herstellungsmethode für dieses $C_{15}$-Triphenylphosphoniumsalz die Umsetzung des tertiären Alkohols der Formel IIa mit Triphenylphosphin-hydrobromid genannt, wobei das Hydrobromid in einem Überschuß von 17 mol-% eingesetzt wird.

Nachteilig an den Verfahren gemäß dem Stand der Technik ist also, daß in jedem Fall für die Umsetzung der tertiären Alkohole der Formel II zu den entsprechenden Triphenylphosphoniumbromiden mit erheblichen überschüssen an teueren und verfahrenstechnisch nicht unproblematischen Halogenierungsmitteln gearbeitet werden muß. Im Falle der Herstellung von Astaxanthin kommt als weiteres Problem die geringe Stabilität entsprechenden Allylbromids hinzu.

Es war daher die Aufgabe der Erfindung, ein Verfahren zur Herstellung von Canthaxanthin und Astaxanthin zu entwickeln, bei dem die Nachteile des Standes der Technik nicht auftreten.

Es wurde nun überraschenderweise gefunden, daß die tertiären Alkohole der Formel II durch Behandlung mit Trifluoressigsäure glatt in die neuen Trifluoracetate der Formel III und diese durch anschließende Umsetzung mit Triphenylphosphin in die Phosphoniumsalze der Formel IV überführt werden können. Das praktische Vorgehen ist bei beiden Edukten identisch, was einen weiteren erheblichen technischen Vorteil für dieses neue Verfahren bedeutet.

Gegenstand der Erfindung ist daher ein Verfahren zur Herstellung von Canthaxanthin (Ia) und Astaxanthin (Ib) der allgemeinen Formel I

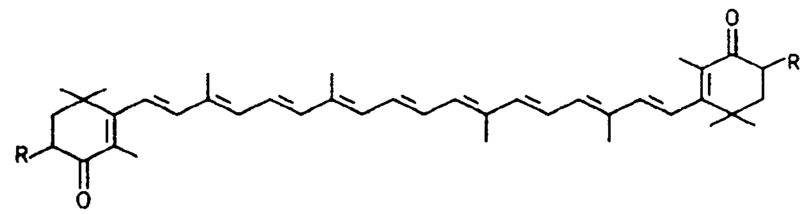

in der R für H (a) oder OH (b) steht, das dadurch gekennzeichnet ist, daß man

A. einen tertiären Alkohol der allgemeinen Formel II

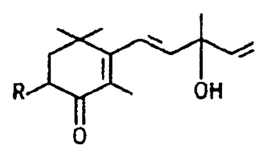

(II)

in der R für H (a) oder OH (b) steht, mit Trifluoressigsäure umsetzt,

B. das erhaltene neue Trifluoracetat der allgemeinen Formel III

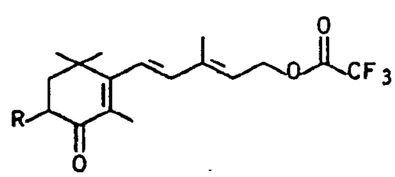

(III)

mit Triphenylphosphin umsetzt,

C. das erhaltene neue Triphenylphosphoniumtrifluoracetat der allgemeinen Formel IV

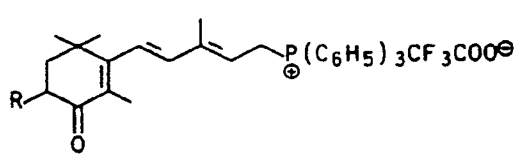

(IV)

in der R für H (a) oder OH (b) steht,

mit 2,7-Dimethyl-2,4,6-octatrien-1,8-dial unter den Bedingungen einer Wittigsynthese umsetzt.

Gegenstand der Erfindung sind außerdem die neuen Zwischenprodukte der Formeln III und IV, die das vorteilhafte neue Verfahren ermöglichen.

Die Umsetzung der tertiären Alkohole der Formel II mit Trifluoressigsäure wird im allgemeinen in einem inerten organischen Lösungsmittel, wie Methylenchlorid, 1,2-Dichlorethan oder Essigsäureethylester bei Temperaturen von etwa 0°C bis +50°C, vorzugsweise 10-30°C, insbesondere bei Raumtemperatur, durchgeführt.

Es werden hierbei nur 1,0-1,05 Äquivalente an Trifluoressigsäure, bezogen auf die Alkohole der Formel II benötigt. Man erhält in glatter Reaktion und in guten Ausbeuten die neuen Trifluoracetate der Formel III, ohne daß die sekundäre Hydroxygruppe von IIIb verestert wird und ohne Gefahr, daß das α-Ketol ins Diosphenol umlagert wird.

Die rohen Trifluoracetate der Formel III können direkt zu den Phosphonium-salzen der Formel IV weiterverarbeitet werden, beispielsweise durch Erhitzen mit einem Triarylphosphin, insbesondere Triphenylphosphin, in einem inerten organischen Lösungsmittel wie Toluol, Essigester oder Methylenchlorid. Besonders einfach läßt sich diese Umsetzung durchführen, indem man die rohen Trifluoracetate in Substanz ohne Zusatz eines Lösungsmittels mit Triphenylphosphin erhitzt.

Das Triphenylphosphin verwendet man hierbei im allgemeinen in Mengen von 1,0 bis 1,2 Mol pro Mol Trifluoracetat der Formel III. Die Umsetzung erfolgt im allgemeinen bei Temperaturen von Raumtemperatur bis Rückflußtemperatur des verwendeten Lösungsmittels, vorzugsweise bei Temperaturen von etwa 50 bis 100°C. Die Reaktionszeit beträgt beispielsweise bei der Durchführung der Umsetzung ohne Lösungsmittel im Temperaturbereich von 80 bis 100°C etwa 15 bis 30 Minuten.

Das so erhaltene rohe Phosphoniumsalz der Formel IV kann durch Fällung aus einem geeigneten organischen Lösungsmittel, wie Methyl-tert.-butylether (MTB) oder Diisopropylether, gereinigt werden.

Aus den Phosphoniumsalzen der Formel IVa bzw. IVb erhält man durch Umsetzung mit dem $C_{10}$-Dialdehyd 2,7-Dimethyl-2,4,6-octatrien-1,8-dial unter den für solche Wittig-Kondensationen typischen Reaktionsbedingungen die Carotinoide Canthaxanthin Ia bzw. Astaxanthin Ib in guten Ausbeuten.

Bezüglich näherer Einzelheiten über die Reaktionsbedingungen für Wittig-Reaktionen verweisen wir beispielsweise auf J. Org. Chem. 47 (1982) Seiten 2130-2134 und Helv. Chim. Acta 64 (1981) Seiten 2436ff.

Die folgenden Beispiele sollen das erfindungsgemäße Verfahren erläutern.

Beispiel 1

A. Herstellung von 5-[2,6,6-Trimethyl-3-oxo-1-cyclohexen-1-yl]-3-methyl-2,4-pentadien-1-ol-trifluoroacetat (IIIa)

10,0 g (42,7 mmol) 5-[2,6,6-Trimethyl-3-oxo-1-cyclohexen-1-yl]-3-methyl-3-hydroxy-1,4-pentadien (IIa) wurden in 40 ml Methylenchlorid gelöst und hierzu bei 0°C 4,4 ml (43,2 mmol) Trifluoressigsäure (D 1,12) zugetropft. Anschließend ließ man das Reaktionsgemisch auf Raumtemperatur (RT) kommen und rührte 3 Stunden (h) bei RT nach.

Danach wurde der Ansatz auf Wasser gegossen. Die organische Phase wurde abgetrennt und die Wasserphase mit Methylenchlorid nachextrahiert. Die vereinigten organischen Phasen wurden mit verdünnter Natriumhydrogencarbonatlösung gewaschen und über Natriumsulfat getrocknet. Nach dem Abziehen des Lösungsmittels am Rotationsverdampfer erhielt man 12 g des Trifluoracetats IIIa als rötlich gefärbtes Öl. IR-Spektrum (Film): 1784 $cm^{-1}$ (vs), 1665 $cm^{-1}$ (s), 1217 $cm^{-1}$ (vs), 1168 $cm^{-1}$ (vs).

B. Herstellung von
5-[2,6,6-Trimethyl-3-oxo-1-cyclohexen-1-yl]-3-methyl-2,4-pentadien-1-yl-triphenylphosphonium-trifluoracetat (IVa)

Das rohe Trifluoracetat IIIa wurde zusammen mit 9,5 g Triphenyl-phosphin 30 Minuten (min) im vorgeheizten Ölbad bei einer Bad temperatur von 100-120°C gerührt. Man ließ den Rückstand abkühlen und löste das rohe Phosphoniumsalz in Methylenchlorid auf. Durch Zutropfen dieser Lösung zu Methyl-tert.-butylether (MTB) fiel das Triphenylphosphoniumsalz IVa in Form weißer Kristalle aus. Fp.: 150-154°C

| | | | | | |
|---|---|---|---|---|---|
| Berechnete Werte (für $C_{35}H_{36}PO_3F_3$): | | | | | |
| | C 71,0 % | H 6,1 % | P 5,2 % | O 8,1 % | F 9,6 % |
| Analytisch gefundene Werte: | | | | | |
| | C 70,6 % | H 5,9 % | P 5,1 % | | F 9,5 % |

C. Herstellung von Canthaxanthin (Ia)

5,3 g des Phosphoniumsalzes IVa und 500 mg 2,7-Dimethyl-2,4,6-octatrien-1,8-dial wurden in 25 ml

Methylenchlorid gelöst. Man kühlte auf 0°C ab, tropfte zu der Mischung 1,6 g einer 30 %igen methanolischen Lösung von Natriummethylat zu, ließ auf RT kommen und rührte 2 h nach Danach wurde der Ansatz auf Wasser gegossen. Die organische Phase wurde abgetrennt und die Wasserphase mit Methylenchlorid nachextrahiert. Die vereinigten organischen Phasen wurden über Natriumsulfat getrocknet und am Rotationsverdampfer eingeengt. Der Rückstand wurde mit 20 ml Methanol aufgenommen und 30 min unter Rückfluß zum Sieden erhitzt, dann auf 0°C abgekühlt und das ausgefallene Canthaxanthin abfiltriert.

Auswaage: 1,1 g Canthaxanthin

Durch säulenchromatographische Reinigung der Mutterlauge wurden nochmals 0,4 g Canthaxanthin gewonnen.

Beispiel 2

A. Herstellung von
5-[2,6,6-Trimethyl-3-oxo-4-hydroxy-1-cyclohexen-1-yl]-3-methyl-2,4-pentadien-1-ol-trifluoroacetat (IIIb)

8 g (32 mmol) 5-[2,6,6-Trimethyl-3-oxo-4-hydroxy-1-cyclohexen-1-yl]-3-methyl-3-hydroxy-1,4-pentadien (IIb) wurden in 25 ml Methylenchlorid gelöst. Bei 0°C tropfte man 3,7 g (32,5 mmol) Trifluoressigsäure zu. Man ließ auf RT kommen und rührte 5 h bei RT nach. Dann wurde der Ansatz auf verdünnte Natriumhydrogencarbonatlösung gegossen. Die organische Phase wurde abgetrennt und die Wasserphase mit Methylenchlorid nachextrahiert. Die vereinigten organischen Phasen wurden mit verdünnter Hydrogencarbonatlösung gewaschen und über Natriumsulfat getrocknet. Nach dem Abziehen des Lösungsmittels am Rotationsverdampfer erhielt man 9-10 g des Trifluoracetats IIIb als rohes Öl. IR-Spektrum (Film): 3500 $cm^{-1}$ (breit), 1784 $cm^{-1}$ (vs), 1670 $cm^{-1}$ (s), 1221 $cm^{-1}$ (s), 1168 $cm^{-1}$ (vs), 1149 $cm^{-1}$ (vs).

B. Herstellung von
5-[2,6,6-Trimethyl-3-oxo-4-hydroxy-1-cyclohexen-1-yl]-3-methyl-2,4-pentadien-1-yl-triphenylphosphonium-trifluoracetat (IVb)

1,3 g des erhaltenen rohen Trifluoracetats IIIb wurden mit 1,0 g Triphenylphosphin 30 min im vorgeheizten Ölbad bei einer Badtemperatur von 100°C gerührt. Man ließ den Rückstand abkühlen, löste das rohe Phosphoniumsalz in Essigester auf und fällte durch Zutropfen dieser Lösung zu MTB wieder aus. Der Niederschlag wurde abgetrennt und unter vermindertem Druck getrocknet. Man erhielt 1,3-1,4 g des Triphenylphosphoniumsalzes IVb als hellgelben Schaum.

Durch nochmaliges Auflösen in Essigsäureethylester und Zutropfen von MTB wurde das Phosphoniumsalz IVb in Form farbloser Kristalle erhalten. Fp.: 147-148°C, berechneter Fluorwert: 9,4 %, analytisch bestimmter Fluorwert: 9,5 %.

C. Herstellung von Astaxanthin (Ib)

8,4 g des kristallinen Phosphoniumsalzes IVb und 760 mg 2,7-Dimethyl-2,4,6-octatrien-1,8-dial wurden in 33 ml Methylenchlorid gelöst. Man kühlte auf 0°C ab, tropfte zu der Mischung 2,24 g einer 30 %igen methanolischen Lösung von Natriummethylat zu, ließ die Temperatur auf RT ansteigen und rührte noch 3 1/2 h nach. Danach wurde das Reaktionsgemisch auf Wasser gegossen. Die organische Phase wurde abgetrennt und die Wasserphase mit Methylenchlorid nachextrahiert. Die vereinigten organischen Phasen wurden mit Wasser gewaschen, über Natriumsulfat getrocknet und am Rotationsverdampfer eingeengt. Durch säulenchromatographische Reinigung des Rohproduktes wurde sauberes Astaxanthin gewonnen.

Auswaage: 2,3 g Astaxanthin.

## Patentansprüche

1. Verfahren zur Herstellung von Canthaxanthin (Ia) und Astaxanthin (Ib) der allgemeinen Formel I

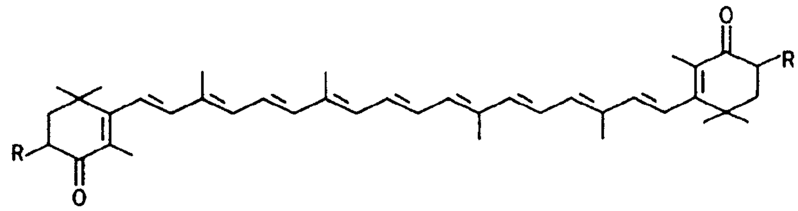

in der R für H (a) oder OH (b) steht, dadurch gekennzeichnet, daß man

A. einen tertiären Alkohol der allgemeinen Formel II

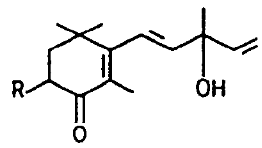

(II)

in der R für H (a) oder OH (b) steht, mit Trifluoressigsäure umsetzt,

B. das erhaltene neue Trifluoracetat der allgemeinen Formel III

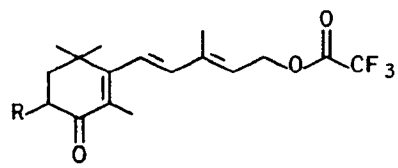

(III)

mit Triphenylphosphin umsetzt,

C. das erhaltene neue Triphenylphosphoniumtrifluoracetat der allgemeinen Formel IV

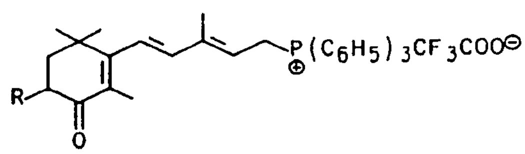

(IV)

in der R für H (a) oder OH (b) steht,

mit 2,7-Dimethyl-2,4,6-octatrien-1,8-dial unter den Bedingungen einer Wittigsynthese umsetzt.

2. Verbindungen der Formel III

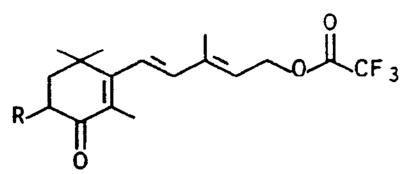

(III)

in der R für H (a) oder OH (b) steht.

3. Verbindungen der Formel IV

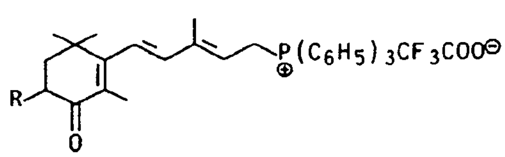

(IV)

in der R für H (a) oder OH (b) steht.